# EUROPEAN PATENT APPLICATION

(11) **EP 4 847 037 A1**
(43) Date of publication of application: **02.12.2026**
(21) Application number: 25180015.7
(22) Date of filing: 30.05.2025
(51) Int. Cl.: A61K 39/00, A61K 39/385, A61P 35/00, C07K 14/005

(54) **VIRUS-LIKE PARTICLES DISPLAYING HER-2 EXTRACELLULAR DOMAIN**

(71) Applicant: Expres2ion Biotechnologies ApS, 2970 Hørsholm (DK)
(72) Inventor: Rasmussen, Christina Alberg Jo, 3660 Stenløse (DK); Nielsen, Ida Busch, 2720 Vanløse (DK); Søgaard, Teit Max Moscote, 2980 Kokkedal (DK)
(74) Representative: Inspicos P/S

(57) **Abstract**

Disclosed is a variant of HER-2, which includes an N-terminal catcher domain and the HER-2 extracellular domain. Also disclosed is VLP monomer subunits as well as a VLP, which comprises HER-2 coupled via an isopeptide bond to a self-assembling virus coat protein.

## Description

### FIELD OF THE INVENTION

The present invention relates to therapy of cancer expressing the HER-2 gene, in particular breast cancer. More specifically, the invention relates to active specific immunotherapy targeting HER-2 and to immunogenic agents suitable for this purpose.

### BACKGROUND OF THE INVENTION

HER-2 is a tumour-associated antigen, which is overexpressed on some tumour cells and to a lesser extent expressed on normal cells, which makes HER-2 a recognized target for therapeutic cancer vaccines. Thus, HER-2 is a self-protein. Most self-proteins in the body, such as HER-2, are not antigenic because of self-tolerance establish during foetal life, a process in which self-reacting cytotoxic T-lymphocytes and autoantibody-producing B-lymphocytes are deleted or inactivated "centrally" in the thymus and "peripherally" in the spleen and lymph nodes when exposed to self-antigens in a non-danger signal context. Conversely, any self-protein, that for some reason has not been exposed to the immune system in the thymus, may later trigger an immune response, since central self-tolerance has not been developed. This may include normal proteins that are well sequestered from the immune system, proteins that are normally produced in extremely small quantities, or proteins whose structure is modified due to mutation.

The definition of HER-2-positive breast cancer is based on patients expected to benefit from immunotherapy (e.g. trastuzumab and pertuzumab) per the inclusion criteria used in pivotal trials. Low expressing HER-2 (IHC 1+, or 2+ and no gene expression/FISH -) may be a distinct, clinically relevant breast cancer population warranting reassessment of traditional diagnostic and therapeutic paradigms (Prat et al., 2022). Up to 85% of patients have traditionally defined HER-2-negative breast cancer and are ineligible for current anti-HER-2 directed therapies; however, 50% of patients with HER-2-negative BC nevertheless exhibit low levels of HER-2 expression.

HER-2-low patients have been extensively studied in multiple HER-2 vaccine trials over the last two decades. Overall, more than 1.000 patients with HER-2-low expressing breast cancer were exposed to various HER-2 vaccines with and without adjuvant in these trials, and no serious safety concerns were raised with only rare incidence of grade 3 toxicities. Most importantly, a trend for efficacy was observed by improved disease-free survival (DFS) in this patient population. This means that preferentially if not exclusively patients were studied after the first line of therapy. Therefore, the overall risk-benefit ratio of including HER-2-low patients in HER-2 vaccine trials seems favourable.

However, there is currently no effective active, specific immunotherapy approach that satisfactorily target HER-2.

### OBJECT OF THE INVENTION

It is an object of embodiments of the invention to provide vaccine agents and related precursors thereof, which can induce clinically relevant immune responses against HER-2, and thereby provide a therapeutic approach that targets HER-2, in particular in patients that exhibit a HER-2-low phenotype.

### SUMMARY OF THE INVENTION

The extracellular domain (ECD) of human Receptor tyrosine-protein kinase erbB-2 protein (hereafter named HER-2_ECD) was expressed in Drosophila Melanogaster S2 cells as a fusion protein with an N-terminal "MoonCake" domain which catalyses spontaneous and covalent crosslinking to a "RumTrunk" peptide displayed on the N-terminus of each of 180 capsomers which self-assemble to a Virus Like Particle (VLP) derived from the Acinetobacter phage AP205. When purified VLP and fusion protein are mixed, the crosslinking results in the decoration of the VLP surface with fusion protein. This creates a vaccine candidate for HER-2 positive breast cancer and other HER-2 expressing cancers.

In particular, the extracellular domain (ECD) of HER-2 was expressed in *Drosophila Melanogaster* Schneider 2 cells ("S2 cells") as an N-terminal fusion with the Mooncake enzyme (also termed a catcher domain herein. The fusion protein was purified using a C-terminal affinity tag (C-tag, Thermo Fisher) and used as a HER-2 positive breast cancer vaccine.

To reduce noise when measuring antibodies raised in mice in an ELISA, an ELISA control protein with no extraneous sequences had to be provided. A construct without the N-terminal catcher domain was hence prepared from transfected S2 cells.

Surprisingly, the transfected cells expressed significantly less of the control protein than of the fusion protein, suggesting the existence of an expression enhancing effect of the N-terminal fusion partner, in particular the catcher domain. Consequently, a series of constructs were prepared to test fusion partners, including catcher/bait domains and affinity tags and locations of these on HER-2.

So, in a first aspect the present invention relates to a variant of HER-2 protein, which comprises the following amino acid sequence elements in N to C direction:
a. a catcher domain comprising or consisting of amino acid sequence SEQ ID NO: 3: 
GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHI
b. optionally one or more spacer amino acids,
c. a HER-2 extracellular domain, and
d. optionally an affinity tag.

In a second aspect, the invention relates to a VLP (virus-like particle) monomer subunit, which comprises the variant of the 1^{st} aspect of the invention as well as any embodiments thereof disclosed herein, wherein said variant is linked to a polypeptide comprising in the N to C direction the following sub-elements a and b:
a. a bait domain consisting of amino acid sequence SEQ ID NO: 6:
   GNPLIVMVND TTKVK, and
b. a VLP monomer coat protein,
wherein a and b are joined via linker amino acid residues or a bond.

In a 3^{rd} aspect, the present invention relates to a virus like particle (VLP) composed of components i and ii:
i) a plurality of VLP monomer subunits of the 1^{st} aspect of the invention as well as any embodiments thereof disclosed herein, and
ii) a plurality of sub-elements a and b defined in respect of the 2^{nd} aspect of the invention as well as any embodiments thereof disclosed herein.

In a 4^{th} aspect of the invention is provided a composition comprising the VLP of the 3^{rd} aspect of the invention as well as any embodiment thereof disclosed herein in admixture with a pharmaceutically acceptable vehicle, carrier or excipient.

In a 5^{th} aspect, the present invention relates to a nucleic acid molecule encoding the variant of the 1^{st} aspect of the invention as well as any embodiments thereof disclosed herein.

In a 6^{th} aspect, the present invention relates to an expression vector comprising and being capable or expressing the nucleic acid molecule of the 5^{th} aspect of the invention as well as any embodiments thereof disclosed herein.

In a 7^{th} aspect, the present invention relates to an insect cell or cell line, which is transfected with the vector of the 6^{th} aspect of the invention as well as any embodiments thereof disclosed herein.

In an 8^{th} aspect, the present invention relates to a method of preparing the variant according of the 1^{st} aspect of the invention as well as any embodiments thereof described herein, the method comprising culturing the insect cell or cell line of the 7^{th} aspect of the invention, as well as any embodiments thereof described herein, under conditions that facilitate expression of the nucleic acid of the 5^{th} aspect of the invention as well as any embodiments thereof described herein, and subsequently recovering and optionally purifying the variant from the culture medium.

In a 9^{th} aspect, the present invention relates to a method of preparing the VLP according to the 3^{rd} aspect of the present invention as well as any embodiments thereof described herein, the method comprising admixing the variant according to the 1^{st} aspect of the invention as well as any embodiment thereof described herein with self-assembled VLP precursors consisting of 80 sub-elements a and b defined in respect of the 2^{nd} aspect of the invention and any embodiment thereof disclosed herein under conditions that facilitate coupling between catcher and bait domains via isopeptide bond formation.

Finally, in a 10^{th} aspect, the present invention relates to a method of immunizing a human subject so as to induce an immune response against HER-2, comprising administration of an immunologically effective amount of the VLP of the 3^{rd} aspect of the present invention as well as any embodiment thereof, or the composition of the 4^{th} aspect of the present invention as well as any embodiment thereof described herein, to said human subject.

### LEGENDS TO THE FIGURE

Fig. 1: MoonCake-HER-2_ECD capture data.
   A: Capture chromatogram.
   B: Coomassie stained, reduced or non-reduced SDS-PAGE analysis of fractions (left panel) and loading pattern (right panel).
   MoonCake-HER-2_ECD was captured on Capture Select C-tagXL resin.
Fig. 2: IMAC capture chromatogram and Coomassie stained, reduced SDS-PAGE analysis of fractions of HER-2_ECD-6xHIS protein.
   A: IMAC capture chromatogram.
   B: Coomassie stained gel (left panel) and loading pattern (right panel).
Fig. 3: Prediction by SignalP 6.0 of secretion signal cleavage from HER-2_ECD-Strep tag protein.
Fig. 4: Result of N-terminal sequencing of HER-2_ECD-Strep tag protein.
Fig. 5: Schematic representation of N-terminally extended HER-2_ECD constructs.
Fig. 6: Twin-Strep Capture of Twin-Strep-HER-2_ECD.
   A: Chromatogram.
   B: Coomassie stained SDS-PAGE analysis of elution fractions (left panel) and loading pattern (right panel).
Fig. 7: Capture of hCPD-HER-2_ECD-C using C tag.
   A: Chromatogram.
   B: Coomassie stained SDS-PAGE analysis of elution fractions (left panel) and loading pattern (right panel).

### DETAILED DISCLOSURE OF THE INVENTION

### Definitions

"HER-2" is the human protein encoded by the ERBB2 gene. The full-length protein includes an extracellular domain (ECD), a transmembrane region, and an intracellular domain. For the purposes of the present invention, the transmembrane region and the intracellular domain are irrelevant. HER-2 exists in a number of allelic variants, the ECD of which all exhibit a very high sequence identity with SEQ ID NO: 2 (which is an exemplary, and preferred, amino acid sequence of the HER-2 ECD).

A "variant" of HER-2 is a variant disclosed herein, which at least includes an N-terminally added amino acid sequence, typically a catcher domain.

A "cathcher domain" is one part of a 2 part system, which also contains a "bait domain" - both are amino acid sequences. The two amino acid sequences of the catcher and bait have the ability to link via a isopeptide bond.

An "affinity tag" is an amino acid sequence (typically relatively short), which exhibits a high (even specific) affintity for a capture agent; details are provided below.

### Specific embodiments of the invention

### 1^{st} aspect of the invention and its embodiments

This aspect relates toa variant of HER-2 protein, which comprises the following amino acid sequence elements in N to C direction:
a. a catcher domain comprising or consisting of amino acid sequence SEQ ID NO: 3: 
GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHI
b. optionally one or more spacer amino acids,
c. a HER-2 extracellular domain ("HER-2 ECD"), and
d. optionally an affinity tag.

In the practice of the present invention, a catcher domain is used in the variants described herein, but as is clear from the examples, it appears to be the presence of an N-terminal fusion partner of a certain size that provides the surprising advantage of increasing expression levels of HER-2 ECD. Hence, also provided as an invention in its own right is a variant of HER-2 ECD, which comprises a fusion partner, which is not a catcher domain, in the N-terminus. Otherwise, all disclosure below apply *mutatis mutandis* to such a construct, where the catcher domain is substituted with such other fusion partners. The length of such fusion partners are typically in the range of 50-150 amino acid residues.

The variant is in preferred embodiments essentially free from transmembrane and intracellular parts of HER-2; this means that the variant is primarily a variant of HER-2 ECD with a few additional features.

The HER-2 extracellular domain in particular interesting embodiments comprises or consists of amino acid sequence SEQ ID NO: 2: 
STQVCTGTDM KLRLPASPET HLDMLRHLYQ GCQVVQGNLE LTYLPTNASL SFLQDIQEVQ
GYVLIAHNQV RQVPLQRLRI VRGTQLFEDN YALAVLDNGD PLNNTTPVTG ASPGGLRELQ
 
LRSLTEILKG GVLIQRNPQL CYQDTILWKD IFHKNNQLAL TLIDTNRSRA CHPCSPMCKG
SRCWGESSED CQSLTRTVCA GGCARCKGPL PTDCCHEQCA AGCTGPKHSD CLACLHFNHS
GICELHCPAL VTYNTDTFES MPNPEGRYTF GASCVTACPY NYLSTDVGSC TLVCPLHNQE
VTAEDGTQRC EKCSKPCARV CYGLGMEHLR EVRAVTSANI QEFAGCKKIF GSLAFLPESF
DGDPASNTAP LQPEQLQVFE TLEEITGYLY ISAWPDSLPD LSVFQNLQVI RGRILHNGAY
SLTLQGLGIS WLGLRSLREL GSGLALIHHN THLCFVHTVP WDQLFRNPHQ ALLHTANRPE
DECVGEGLAC HQLCARGHCW GPGPTQCVNC SQFLRGQECV EECRVLQGLP REYVNARHCL
PCHPECQPQN GSVTCFGPEA DQCVACAHYK DPPFCVARCP SGVKPDLSYM PIWKFPDEEG
ACQPCPINCT HSCVDLDDKG CPAEQRASPL T,
 or an amino acid sequence, which has a sequence identity of at least 96% in a pairwise global alignment with SEQ ID NO: 2. It has turned out that HER-2 ECD exists naturally as allelic variants that exhibit minor sequence variations compared to SEQ ID NO: 2, meaning that deviation in sequence form SEQ ID NO: 2 is possible, in particular if the deviation provides for the sequence of an allelic variant. However, in preferred embodiments, the HER-2 extracellular domain consists of the amino acid sequence SEQ ID NO: 2.

In preferred embodiments of the 1^{st} aspect, the variant (also) include the one or more spacer amino acid residues referred to above; these spacer amino acids that separate the N-terminal fusion partner (which is normally the catcher domain are glycine residues, preferably in the form of 2 glycine residues, i.e. a di-glycine linker.

Also, in preferred embodiments of the 1^{st} aspect of the invention, the variant further includes the affinity tag, preferably as the C-terminal sequence element of the variant. The affinity tag can be any suitable tag (i.e. a relatively short amino acid stretch, which does not interfere with the selected expression system for producing the variant of the 1^{st} aspect) that provides for chromatographic affinity purification via reversible binding to a binding partner. A non-exhaustive list of affinity tags is provided here (where no specific binding partner is indicated, the tag binds an antibody):
ALFA-tag (SRLEEELRRRLTE, SEQ ID NO: 13); AviTag (GLNDIFEAQKIEWHE, SEQ ID NO: 14) allowing biotinylation by the enzyme BirA so as to isolate with streptavidin; C-tag (EPEA, SEQ ID NO: 4); Calmodulin-tag (KRRWKKNFIAVSAANRFKKISSSGAL, SEQ ID NO: 15), which binds calmodulin; iCapTag^{™} (MIKIATRKYLGKQNVYGIGVERDHNFALKNGFIAHN, SEQ ID NO: 16) a self-removing peptide-based tag; polyglutamate tag (EEEEEE, SEQ ID NO: 17), which binds anion-exchange resin; polyarginine tag (RRRRRXXXX, SEQ ID NO: 18, where X is R or absent), which bind cation-exchange resins; E-tag (GAPVPYPDPLEPR, SEQ ID NO: 19); FLAG-tag (DYKDDDDK, SEQ ID NO: 20); HA-tag, a peptide from hemagglutinin recognized by an antibody (YPYDVPDYA, SEQ ID NO: 21), His-tag (HHHHHXXXXX, SEQ ID NO: 22), which is bound by a nickel or cobalt chelate; Gly-His-tags are N-terminal His-Tag variants bind to immobilised metal cations but can also be activated via azidogluconoylation to enable click-chemistry; Myc-tag (EQKLISEEDL, SEQ ID NO: 23); NE-tag (TKENPRSNQEESYDDNES, SEQ ID NO: 24); Rho1D4-tag (TETSQVAPA, SEQ ID NO: 25); S-tag (KETAAAKFERQHMDS, SEQ ID NO: 26); SBT-tag (MDEKTTGWRGGHVVEGLAGELEQLRARLEHHPQGQREP, SEQ ID NO: 27), which binds streptavidin; Softag 1 (SLAELLNAGLGGS, SEQ ID NO: 28); Spot-tag (PDRVRAVSHWSS, SEQ ID NO: 29); Strep-tag (WRHPQFGG, SEQ ID NO: 30) and Strep tag II (WSHPQFEK, SEQ ID NO: 31) both binding streptavidin; T7-tag, an epitope tag derived from the T7 major capsid protein of the T7 gene (MASMTGGQQMG, SEQ ID NO: 32); TC tag (CCPGCC, SEQ ID NO: 33) recognized by FlAsH and ReAsH biarsenical compounds; Ty tag (EVHTNQDPLD, SEQ ID NO: 34); V5 tag (GKPIPNPLLGLDST, SEQ ID NO: 35); VSV-tag (YTDIEMNRLGK, SEQ ID NO: 36); and Xpress tag (DLYDDDDK, SEQ ID NO: 37).

The preferred affinity tag is the C-tag (SEQ ID NO: 4). At any rate, the C-terminal amino acid residue in the variant is preferably the C-terminal amino acid residue in the affinity tag, e.g. the C-terminal A in SEQ ID NO: 4.

As for the N-terminal amino acid residue of the variant of the 1^{st} aspect of the invention, this is in preferred embodiments the N-terminal amino acid of the catcher domain.

A particularly preferred variant according to the 1^{st} aspect of the invention consists of a C-terminal catcher domain followed directly by a di-glycine linker followed directly by a HER-2 ECD, and followed directly by an N-terminal affinity tag. A particularly important variant of the 1^{st} aspect of the present invention consists of the amino acid sequence SEQ ID NO: 1:

GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHIGGSTQV
CTGTDMKLRL PASPETHLDM LRHLYQGCQV VQGNLELTYL PTNASLSFLQ DIQEVQGYVL
IAHNQVRQVP LQRLRIVRGT QLFEDNYALA VLDNGDPLNN TTPVTGASPG GLRELQLRSL
TEILKGGVLI QRNPQLCYQD TILWKDIFHK NNQLALTLID TNRSRACHPC SPMCKGSRCW
GESSEDCQSL TRTVCAGGCA RCKGPLPTDC CHEQCAAGCT GPKHSDCLAC LHFNHSGICE
LHCPALVTYN TDTFESMPNP EGRYTFGASC VTACPYNYLS TDVGSCTLVC PLHNQEVTAE
DGTQRCEKCS KPCARVCYGL GMEHLREVRA VTSANIQEFA GCKKIFGSLA FLPESFDGDP
ASNTAPLQPE QLQVFETLEE ITGYLYISAW PDSLPDLSVF QNLQVIRGRI LHNGAYSLTL
QGLGISWLGL RSLRELGSGL ALIHHNTHLC FVHTVPWDQL FRNPHQALLH TANRPEDECV
GEGLACHQLC ARGHCWGPGP TQCVNCSQFL RGQECVEECR VLQGLPREYV NARHCLPCHP
ECQPQNGSVT CFGPEADQCV ACAHYKDPPF CVARCPSGVK PDLSYMPIWK FPDEEGACQP
CPINCTHSCV DLDDKGCPAE QRASPLTEPE A

In SEQ ID NO: 1 shown above, boldface indicates the catcher domain having SEQ ID NO: 3, underline indicates the preferred di-glycine linker, and italics indicate the preferred C-tag SEQ ID NO: 4, whereas the remaining sequence is the HER-2 ECD SEQ ID NO: 2.

The variant of HER-2 can as shown herein be obtained by a method comprising expressing polynucleotide(s) encoding it in an S2 cell (or other suitable cell) genetically modified so as to express the polynucleotides; details are provided below.

### 2^{nd} aspect of the invention and its embodiments

The 2^{nd} aspect relates to a VLP (virus-like particle) monomer subunit, which comprises the variant of the 1^{st} aspect of the invention as well as any embodiments thereof disclosed herein, wherein said variant is linked to a polypeptide comprising in the N to C direction the following sub-elements a and b:
a. a bait domain consisting of amino acid sequence SEQ ID NO: 6:
   GNPLIVMVND TTKVK, and
b. a VLP monomer coat protein,
wherein a and b are joined via linker amino acid residues or a bond.

Normally, "a" and "b" are joined via linker amino acid residues, which for instance can be a glycine-rich linker, preferably where the linker amino acid residues constitute amino acid sequence SEQ ID NO: 7: GSGTAGGGSGS

In principle, the VLP monomer coat protein of the subunit can be any VLP monomer coat protein known in the art, preferably any VLP coat protein that self-assembles with identical coat proteins to form a VLP under conditions facilitating such self-assembly. A review relating to the current VLP technology and the types of VLPs (and hence coat proteins) used is provided in Mohsen M.O. and Bachmann M.F. 2022, Cellular & Molecular Immunology **19,** 993-1011; these are in principle all relevant for the purposes of the present invention.

However, the preferred VLP monomer coat protein comprises or consists of amino acid sequence SEQ ID NO: 8:

ANKPMQPITS TANKIVWSDP TRLSTTFSAS LLRQRVKVGI AELNNVSGQY VSVYKRPAPK
PEGCADACVI MPNENQSIRT VISGSAENLA TLKAEWETHK RNVDTLFASG NAGLGFLDPT
AAIVSSDTTA

In preferred embodiments, the VLP monomer subunit is one, wherein the variant and the polypeptide are linked via an isopeptide bond between the bait domain and the catcher domain. The isopeptide bond preferably links a K residue in the catcher domain of the variant and an N or D residue in the bait domain of the polypeptide. This effect is attained if the catcher domain has SEQ ID NO: 3, and the bait domain has SEQ ID NO: 6. Hence it is preferred in the VLP monomers of the 2^{nd} aspect of the invention that the K residue in the catcher domain is residue 32 in SEQ ID NO: 3, corresponding to residue 32 in SEQ ID NO: 1. Further, in the VLP monomer subunit of the 2^{nd} aspect the present invention, preferred embodiments are those where the N or D residue in the bait domain is the N in position 9 in SEQ ID NO: 6, corresponding to the N in position 10 in SEQ ID NO: 5.

In other words, a highly preferred VLP monomer subunit is one where the variant of the first aspect of the invention is linked to the polypeptide of sequence SEQ ID NO: 5:

MGNPLIVMVN DTTKVKGSGT AGGGSGSANK PMQPITSTAN KIVWSDPTRL STTFSASLLR
QRVKVGIAEL NNVSGQYVSV YKRPAPKPEG CADACVIMPN ENQSIRTVIS GSAENLATLK
AEWETHKRNV DTLFASGNAG LGFLDPTAAI VSSDTTA

In turn, this means that the most preferred VLP monomer subunit consists of the variant of SEQ ID NO: 1 linked via an isopeptide bond to the polypeptide of SEQ ID NO: 5, where the isopeptide bond links K in position 32 in SEQ ID NO: 1 with N in position 10 in SEQ ID NO: 5.

### 3^{rd} aspect of the invention and its embodiments

The 3^{rd} aspect relates to a virus like particle (VLP) composed of components i and ii:
i) a plurality of VLP monomer subunits of the 2^{nd} aspect of the present invention as well as any embodiments thereof disclosed herein, and
ii) a plurality of polypeptides each composed of sub-elements a and b defined above in respect of the 2^{nd} aspect of the present invention and its embodiments,
preferably where each of the VLP monomer subunits of i) consists of the above-identified most preferred monomer subunit, and where each of the polypeptides of ii) consists of SEQ ID NO: 5.

The VLP in preferred embodiments is self-assembled and composed of 180 units; hence it is preferred that total number of components i and ii is 180; of this total number, about 50 of are constituted by component i. When the VLP is composed the above identified most preferred monomer subunits in i) and when each of the polypeptides in ii) consists of SEQ ID NO: 5, then it has been observed by the inventors that steric hindrance allows for incorporation of about 50 of the most preferred monomer subunits, leaving the reaming part of the VLP to be comstituted by the polypeptide having SEQ ID NO: 5. In turn, this provides for a VLP, which has a molecular weight of about 7 MDa. It will be understood that while the 180 units is a non-variable number due to the self-assembling characteristics of VLPs that contain SEQ ID NO: 5, the exact proportion of the 180 units, which is constituted by the most preferred VLP monomer subunits will vary between the monomers; the number 50 is hence an observed average.

Hence, the most preferred VLP of the present invention is composed of components i and ii:
i) a plurality of VLP monomer subunits, where each of the VLP monomer subunits of i) consists of the variant of amino acid sequence SEQ ID NO: 1 linked via an isopeptide bond to the polypeptide of SEQ ID NO: 5, where the isopeptide bond links K in position 32 in SEQ ID NO: 1 with N in position 10 in SEQ ID NO: 5, and
ii) a plurality of polypeptides each consisting of SEQ ID NO: 5,
wherein the sum of the number of VLP monomer subunits in i) and number of polypeptides in ii) is 180, or which about 50 are VLP monomer subunits.

### 4^{th} aspect of the invention and its embodiments

The 4^{th} aspect relates to a composition comprising the VLP of the 3^{rd} aspect of the invention as well as any embodiment thereof disclosed herein, in admixture with a pharmaceutically acceptable vehicle, carrier, diluent, or excipient. In the general sections below is provided an overview of such pharmaceutically acceptable vehicles, carriers, diluents and excipients. It is noted that the composition of the 4^{th} aspect can comprise any combination of carriers, vehicles, diluent, and excipient, i.e. only one of these 4 species, to of the 4 species, 3 or the species or all 4.

In addition to the VLP, the composition preferably comprises at least one buffering agent, and at least one tonicifying agent. Also these agents are detailed further in the examples and the general sections below.

The composition can conveniently be provided in dry form, preferably in lyophilized form; at the time of use, it is then possible to reconstitute the composition in a suitable solvent, typically an aqueous. As an alternative to the dry form, the composition is in the form of an aqueous composition.

In some embodiments, the composition comprises an immunological adjuvant. Again, a detailed disclosure of immunological adjuvants is provided in general sections below. However, the immunological adjuvant is typically a water-in-oil emulsion adjuvant, preferably in the form of a mineral oil in admixture with a mannide monooleate surfactant. A preferred adjuvant is Montanide ISA 51.

The most preferred composition comprises water in admixture with 3.0 mg/ml of the VLP*, 8.0 mg/ml NaCl, 0.2 mg/ml KCl, 0.2 mg/ml KH₂PO₄, 1.4 mg/ml Na₂PO₄·2H₂O, 1.2 mg/ml tris(hydroxymethyl)aminomethane, and 68.46 mg/ml sucrose.
*) The VLP is composed of components i and ii:
   i) a plurality of VLP monomer subunits, where each of the VLP monomer subunits of consists of the above-identified most preferred monomer subunit, and
   ii) a plurality of polypeptides, which each consists of SEQ ID NO: 5.

### Vaccines of the invention, general teachings

Immunogenic compositions, in particular vaccines, according to the invention (and thus comprising the VLP of the invention) may be prophylactic (i.e. suited to prevent cancer development in particularly susceptible indivuals, such as those that have a genetic dispositon for developing HER-2 positive cancer) or, typically, therapeutic (i.e. to treat disease) or they may be useful for induction of antibody production in animals used for that purpose.

The immunogenic composition of the invention comprises the VLP of the present 3^{rd} aspect fo the invention and embodiments thereof disclosed herein, in admixture with at least one immunological adjuvant and as discussed above the harmaceutically acceptable carrier and/or vehicle and/or diluent and/or excipient; see below for the detailed discussion of immunological adjuvants and other substances in the composition.

Typically, an immunogenic composition of the invention is in the form of a liquid formulation (suitable for injection or ingestion) such as a solution, a suspension, an emulsion, or a suspoemulsion, or in the form of a solid or semisolid formulation, such as a powder, tablet, suppository, pill, gel, cream, or ointment. Conveniently, an immunogenic composition of the invention is contained a unit dose form, such as in freeze-dried form.

Vaccines disclosed herein typically comprise the immunising VLP, usually in combination with "pharmaceutically acceptable carriers", which include any carrier that does not itself induce the production of antibodies harmful to the individual receiving the composition or targeting the protein/pathogen. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles.

Such carriers are well known to those of ordinary skill in the art. Additionally, some carriers may function as immunostimulating agents ("adjuvants"). Furthermore, the antigen or immunogen may be conjugated to a bacterial toxoid, such as a toxoid from diphtheria, tetanus, cholera, H. pylori, etc. pathogen.

A thorough discussion of pharmaceutically acceptable excipients is available in Remington's Pharmaceutical Sciences (Mack Pub. Co., N. J. 1991).

The pharmaceutical compositions disclosed herein thus typically contain an immunological adjuvant, which is commonly an aluminium based adjuvant (including an aluminium salt), an oil-in-water emulsion, a saponin, complete and incomplete Freund's adjuvant, and a cytokine; or one of the other adjuvants described in the following:
Preferred adjuvants to enhance effectiveness of the composition include, but are not limited to:
(1) aluminium salts (alum), such as aluminium hydroxide, aluminium phosphate, aluminium sulfate, etc;
(2) oil-in-water emulsion formulations (with or without other specific immunostimulating agents such as muramyl peptides (see below) or bacterial cell wall components), such as for example (a) MF59 (WO 90/14837; Chapter 10 in Vaccine design: the subunit and adjuvant approach, eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, (c) AddaVax^{™} (a squalene-based oil-in-water nano-emulsion similar to MF59, and (d) Ribi adjuvant system (RAS), (Ribi Immunochem, Hamilton, MT) containing 2% Squalene, 0.2% Tween 80, and one or more bacterial cell wall components from the group consisting of monophosphoryl lipid A (MPL), trehalose dimycolate (TDM), and cell wall skeleton (CWS), preferably MPL + CWS (DetoxTM);
(3) saponin adjuvants such as Stimulon^{™} (Cambridge Bioscience, Worcester, MA) may be used or particles generated therefrom such as ISCOMs (immunostimulating complexes);
(4) Complete Freund's Adjuvant (CFA) and Incomplete Freund's Adjuvant (IFA);
(5) cytokines, such as interleukins (eg. IL-1, IL-2, IL-4, IL-5, IL-6, IL-7, IL-12, etc.), interferons (e.g. gamma interferon), macrophage colony stimulating factor (M-CSF), tumor necrosis factor (TNF), etc.; and
(6) other substances that act as immunostimulating agents to enhance the effectiveness of the composition.

Muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl- L-alanine-2"-2'-dipalmitoyl-sn-glycero-3-hydroxyphosphoryloxy)-ethylamine (MTP-PE), etc.

The preferred adjuvant is a indicated above Montanide ISA 51.

The immunogenic compositions typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the VLP, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individuals to be treated (eg. nonhuman primate, primate, etc.), the capacity of the individual's immune system to synthesize antibodies or generally mount an immune response, the degree of protection desired, the formulation of the vaccine, the treating doctor's assessment of the medical situation, and other relevant factors. It is expected that the amount will fall in a relatively broad range that can be determined through routine trials. However, for the purposes of protein vaccination, the amount administered per immunization is typically in the range between 0.5 µg and 500 mg (however, often not higher than 5,000 µg), and very often in the range between 10 and 200 µg.

The immunogenic compositions are conventionally administered parenterally, eg, by injection, either subcutaneously, intramuscularly, or transdermally/transcutaneously (e.g. as in WO 98/20734). Additional formulations suitable for other modes of administration include oral, pulmonary and nasal formulations, suppositories, and transdermal applications.

Dosage treatment may be a single dose schedule or a multiple dose schedule. The vaccine may be administered in conjunction with other immunoregulatory agents.

### 5^{th} aspect of the invention and its embodiments

The 5^{th} aspect of the present invention relates to a nucleic acid molecule encoding the variant of HER-2 protein of the 1^{st} aspect of the present invention and any embodiments thereof disclosed herein. This nucleic acid molecule is useful in recombinant production of the variant.

Typically the nucleic acid molecule is a DNA molecule, and in preferred embodiments it comprises residues 201-2453 in SEQ ID NO: 10, and in even more preferred embodiments residues 147-2453 in SEQ ID NO: 10; residues 201-542 encodes the catcher domain, residues 543-548 encodes the di-glycine linker, residues 549-2441 encodes the HER-2 ECD, and residues 2442-2453 encodes the C-tag, thus residues 201-2453 encodes the most preferred HER-2 variant disclosed herein. Residues 147-200 encodes the BiP signal peptide coding sequence and thus facilitates export of the preferred construct when expressing in S2 cells.

SEQ ID NO: 10 is the nucleic acid sequence of a preferred expression vector, cf. below.

Generally, the sequence of the nucleic acid molecule of the present invention is codon optimized for expression in S2 cells.

### 6^{th} aspect of the invention and its embodiments

This aspect relates to an expression vector, which comprises and is capable of expressing the nucleic acid molecule according to the 5^{th} aspect of the present invention and the embodiments thereof disclosed herein. The preferred expression vectors of the 6^{th} aspect are capable of being expressed in *Drosophila* S2 cells. They will typically be plasmids. One especially preferred plasmid has the nucleotide sequence SEQ ID NO: 10.

A more general discussion of expression vectors, host cells and expression systems is provided below in the general section

### 7^{th} aspect of the invention and its embodiments

The 7^{th} aspect of the present invention relates to an insect cell or cell line, which is transfected with the expression vector of the 6^{th} aspect of the present invention as well as any embodiment thereof disclosed herein; preferably such an insect cell is stably transfected with the vector, thus facilitating reproducible recombinant production of the expression product. Preferred insect cells are Drosophila cells or cell lines, preferably an S2 cell or cell line.

Genetically modified cells disclosed herein are useful as organisms for producing proteins and polypeptides, in particular the HER-2 variant of the 1^{st} aspect of the invention.

The most preferred cell of the 7^{th} aspect is an insect cell (such as a *Drosophila* S cell), the specific modifications introduced in the HER-2 variant of the 1^{st} aspect are also believed to be relevant in a range of other cell types, such as mammalian cells and fungal cells (including yeast or filamentous fungal cells).

As mentioned above, the preferred genetically modified cell of the 7^{th} aspect is an insect cell, preferably a *Drosophila melanogaster* cell, such as an S2 or S3, or an insect cell such as Sf9, SF21, High5, and C6-36. However, in the event the cell is mammalian, it can be a CHO or HEK cell.

### Useful cells, general teachings

Eukaryotic cells can be in the form of yeasts (such as *Saccharomyces cerevisiae)* and protozoans. Alternatively, the transformed eukaryotic cells are derived from a multicellular organism such as a fungus, an insect cell, or a mammalian cell.

For production purposes, it is advantageous that the genetically modified cell disclosed herein is stably transformed by having the nucleic acids disclosed above stably integrated into its genome, and in certain embodiments it is also preferred that the genetically modified cell secretes or carries on its surface the glycosylated polypeptide disclosed herein, since this facilitates recovery of the polypeptides produced.

As noted above, stably genetically modified cells are preferred - these *i.a.* allows that cell lines comprised of genetically modified cells as defined herein may be established - such cell lines are particularly preferred aspects of the invention.

It is noted that the genetically modified cell of the 7^{th} aspect can be established as a cell line comprising the genetically modified cell; one example of such a cell line is a clonal cell line.

Further details on cells and cell lines are presented in the following:
Techniques for recombinant gene production, introduction into a cell, and recombinant gene expression are well known in the art. Examples of such techniques are provided in references such as Ausubel, Current Protocols in Molecular Biology, John Wiley, 1987-2002, and Greene and Sambrook: "Molecular Cloning: A Laboratory Manual (Fourth Edition)", Cold Spring Harbor Laboratory Press (ISBN-10: 9781936113422).

As used herein, the terms "cell", "cell line", and "cell culture" may be used interchangeably. All of these terms also include their progeny, which is any and all subsequent generations. It is understood that all progeny may not be identical due to deliberate or inadvertent mutations. In the context of expressing a heterologous nucleic acid sequence, "host cell" refers to a eukaryotic cell, and it includes any transformable organism that is capable of replicating a vector or expressing a heterologous gene encoded by a vector. A host cell can, and has been, used as a recipient for vectors or viruses. A host cell may be "transfected" or "transformed," which refers to a process by which exogenous nucleic acid, such as a recombinant protein-encoding sequence, is transferred or introduced into the host cell. A genetically modified cell includes the primary subject cell and its progeny.

Host cells are in the present application derived from eukaryotes, including yeast cells, insect cells, plant cells, and mammalian cells for replication of the vector or expression of part or all of the nucleic acid sequence(s). Numerous cell lines and cultures are available for use as a host cell, and they can be obtained through the American Type Culture Collection (ATCC), which is an organization that serves as an archive for living cultures and genetic materials or from other depository institutions such as Deutsche Sammlung vor Micrroorganismen und Zellkulturen (DSM). An appropriate host can be determined by one of skill in the art based on the vector backbone and the desired result. A plasmid or cosmid, for example, can be introduced into a prokaryote host cell for replication of many vectors or expression of encoded proteins.

Examples of eukaryotic host cells for replication and/or expression of a vector include HeLa, NIH3T3, Jurkat, HEK293, Cos, CHO, Saos, and PC12. Many host cells from various cell types and organisms are available and would be known to one of skill in the art. Similarly, a viral vector may be used in conjunction with a host cell, particularly one that is permissive for replication or expression of the vector.

Some vectors may employ control sequences that allow it to be replicated and/or expressed in both prokaryotic and eukaryotic cells. One of skill in the art would further understand the conditions under which to incubate all of the above described host cells to maintain them and to permit replication of a vector. Also understood and known are techniques and conditions that would allow large-scale production of vectors, as well as production of the nucleic acids encoded by vectors and their cognate polypeptides, proteins, or peptides.

### 8^{th} aspect of the invention and its embodiments

As will be understood from the above and the examples presented below, the variant of HER-2, which forms an important part of the VLPs disclosed herein, can advantageously be produced in insect cells, such as S2 cells. Hence, the 8^{th} aspect relates to a method of preparing the variant of HER-2 protein of the 1^{st} aspect of the present invention and the embodiments thereof disclosed above, the method comprising culturing the insect cell or cell line of the 7^{th} aspect of the present invention and the embodiments thereof disclosed herein under conditions that facilitate expression of the nucleic acid of the 5^{th} aspect of the invention as well as any embodiment thereof disclosed herein, and subsequently recovering and optionally purifying the variant of HER-2 from the culture medium. In the general sections below, more details are provided in respect of such recombinant production.

### 9^{th} aspect of the invention and its embodiments

The 9^{th} aspect of the present invention relates to a method of preparing the VLP of the 3^{rd} aspect of the present invention and the embodiments thereof disclosed herein, the method comprising admixing the variant of HER-2 protein of the 1^{st} aspect of the present invention as well as any embodiment disclosed herein with self-assembled VLP precursors (polypeptides)= consisting of 80 sub-elements "a" and "b" defined above in respect of the 2^{nd} aspect of the present invention and any embodiments therof under conditions that facilitate coupling between catcher and bait domains via isopeptide bond formation. Typically, the variant of HER-2 protein is prepared according to the method of the invention's 8^{th} aspect and any embodiments thereof disclosed herein, i.e. the HER-2 protein is recombinantly produced in insect cells (i.e. an insect cell expression system), preferably in S2 cells. On the other hand, the self-assembled VLP precursors are typically prepared in a bacterial expression system according to methods generally known in the art.

### 10^{th} aspect of the invention and its embodiments

The 10^{th} aspect of the present invention relates to a method of immunizing a human subject so as to induce an immune response against HER-2, comprising administration of 1) an immunologically effective amount of the VLP of the 3^{rd} aspect of the present invention as well as any embodiments thereof disclosed herein, or 2) the composition of the 4^{th} aspect of the present invention as well as any embodiment thereof disclosed herein, to said human subject. In both case, the active principle in the immunizing agent is the most preferred VLP disclosed above.

The treated human subject is typically one suffering from a HER-2 expressing cancer, such as a HER-2 expressing breast cancer.

As for the treatment regiment, the subject is in some embodimens administered a priming administration followed by one or more booster administrations.

### Vaccination , general teachings

As mentioned above, the VLP of of the 3^{rd} aspect of the invention and the embodiments thereof enables the method of the 10^{th} aspect for inducing/enhancing a specific immune response against HER-2..

It is preferred to administer the active principle in both a priming immunization and at least one subsequent booster immunization. Alternatively, the somewhat more recent approach of utilising cluster immunizations (i.e. an immunization scheme where repeated dosages of the immunogen(s) are administered at short intervals in the beginning of the immunization regimen before a memory immune response has been established; this is then followed by delay immunizations that resemble the traditional booster immunization used in a prime-boost immunization regimen).

In some embodiments of the present invention the at least one immunization reduces risk of developing a HER-2 positive cancer - this approach is particularly relevant in individuals that are genectically predisposed of developing such a cancer. However, in the typical embodiments, the at least one immunization treats or ameliorates an existing HER-2 positive cancer.

Immunization routes are typically selected from parenteral routes such as the subcutaneous, intradermal, subdermal, intraperitoneal, intrathecal, and intramuscular routes, or via the oral or oral mucosal routes. See below for details.

To conclude the method of the 10^{th} is normally selected from
a) a method for disease prophylaxis;
b) a method for treatment or amelioration of disease; and
c) a method for antibody production.

Related to the 10^{th} is the VLP of the 3^{rd} aspect of the invention or the composition of the 4^{th} aspect for use in a prophylactic or therapeutice method of the 10^{th} aspect.

The 10^{th} aspect of the invention generally relates to induction of immunity and as such also entails a method that relate to treatment, prophylaxis and amelioration of HER-2 posiive cancer as well as to methods the aim at producing antibodies in a host animal.

Immunization methods entail that the VLP disclosed herein or a composition comprising the VLP is administered to the patient, who typically receives between 0.5 and 5,000 µg of the VLP disclosed herein per administration.

The compositions disclosed herein can induce humoral immunity, so the administration is in some embodiments for the purpose of inducing antibodies specific for HER-2 in a "producer" animal, and wherein said antibodies or B-lymphocytes producing said antibodies are subsequently recovered from the animal to be used in their own right as pharmaceutical, diagnostic or laboratory agents.

The pharmaceutical compositions will comprise a therapeutically effective amount thereof. The term "therapeutically effective amount" or "prophylactically effective amount" as used herein refers to an amount of a therapeutic agent to treat, ameliorate, or prevent HER-2 positive cancer. The precise effective amount for a subject will depend upon the subject's size and health, the nature and extent of the condition, and the therapeutics or combination of therapeutics selected for administration. Thus, it is not useful to specify an exact effective amount in advance. Reference is however made to the ranges for dosages of immunologically effective amounts of VLPs. The effective amount for a given situation can be determined by routine experimentation and is within the judgement of the clinician.

### NUMBERED EMBODIMENTS OF THE INVENTION

In view of the above, the present invention relates in particular to the following numbered embodiments NE1-NE49:
NE1. A variant of HER-2 protein, which comprises the following amino acid sequence elements in N to C direction:
   a. a catcher domain comprising or consisting of amino acid sequence SEQ ID NO: 3: 
GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHI
   b. optionally one or more spacer amino acids,
   c. a HER-2 extracellular domain, and
   d. optionally an affinity tag.
NE2. The variant according to numbered embodiment NE1, which is essentially free from transmembrane and intracellular parts of HER-2.
NE3. The variant according to numbered embodiment NE1 or NE2, wherein the HER-2 extracellular domain comprises or consists of amino acid sequence SEQ ID NO: 2: 
STQVCTGTDM KLRLPASPET HLDMLRHLYQ GCQVVQGNLE LTYLPTNASL SFLQDIQEVQ
GYVLIAHNQV RQVPLQRLRI VRGTQLFEDN YALAVLDNGD PLNNTTPVTG ASPGGLRELQ
LRSLTEILKG GVLIQRNPQL CYQDTILWKD IFHKNNQLAL TLIDTNRSRA CHPCSPMCKG
SRCWGESSED CQSLTRTVCA GGCARCKGPL PTDCCHEQCA AGCTGPKHSD CLACLHFNHS
GICELHCPAL VTYNTDTFES MPNPEGRYTF GASCVTACPY NYLSTDVGSC TLVCPLHNQE
VTAEDGTQRC EKCSKPCARV CYGLGMEHLR EVRAVTSANI QEFAGCKKIF GSLAFLPESF
DGDPASNTAP LQPEQLQVFE TLEEITGYLY ISAWPDSLPD LSVFQNLQVI RGRILHNGAY
SLTLQGLGIS WLGLRSLREL GSGLALIHHN THLCFVHTVP WDQLFRNPHQ ALLHTANRPE
DECVGEGLAC HQLCARGHCW GPGPTQCVNC SQFLRGQECV EECRVLQGLP REYVNARHCL
PCHPECQPQN GSVTCFGPEA DQCVACAHYK DPPFCVARCP SGVKPDLSYM PIWKFPDEEG
ACQPCPINCT HSCVDLDDKG CPAEQRASPL T,
 or an amino acid sequence, which has a sequence identity of at least 96% in a pairwise global alignment with SEQ ID NO: 2.
NE4. The variant according to numbered embodiment 3, wherein the HER-2 extracellular domain consists of SEQ ID NO: 2.
NE5. The variant according to any one of the preceding numbered embodiments, wherein the one or more spacer amino acid residues are present.
NE6. The variant according to numbered embodiment NE5, wherein the spacer amino acids are glycine residues, preferably 2 glycine residues.
NE7. The variant according to any one of the preceding numbered embodiments, wherein the affinity tag is present, preferably as the C-terminal sequence element.
NE8. The variant according to numbered embodiment NE7, wherein the affinity tag has the amino acid sequence EPEA (SEQ ID NO: 4).
NE9. The variant according to numbered embodimentNE7 or NE8, wherein the C-terminal amino acid residue is the C-terminal amino acid residue in the affinity tag, such as the C-terminal A in SEQ ID NO: 4.
NE10. The variant according to any one of the preceding numbered embodiments, wherein the N-terminal amino acid residue is the N-terminal amino acid of the catcher domain.
NE11. The variant according to numbered embodiment NE1, which consists of the amino acid sequence SEQ ID NO: 1: 
GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHIGGSTQV
CTGTDMKLRL PASPETHLDM LRHLYQGCQV VQGNLELTYL PTNASLSFLQ DIQEVQGYVL
IAHNQVRQVP LQRLRIVRGT QLFEDNYALA VLDNGDPLNN TTPVTGASPG GLRELQLRSL
TEILKGGVLI QRNPQLCYQD TILWKDIFHK NNQLALTLID TNRSRACHPC SPMCKGSRCW
GESSEDCQSL TRTVCAGGCA RCKGPLPTDC CHEQCAAGCT GPKHSDCLAC LHFNHSGICE
LHCPALVTYN TDTFESMPNP EGRYTFGASC VTACPYNYLS TDVGSCTLVC PLHNQEVTAE
DGTQRCEKCS KPCARVCYGL GMEHLREVRA VTSANIQEFA GCKKIFGSLA FLPESFDGDP
ASNTAPLQPE QLQVFETLEE ITGYLYISAW PDSLPDLSVF QNLQVIRGRI LHNGAYSLTL
QGLGISWLGL RSLRELGSGL ALIHHNTHLC FVHTVPWDQL FRNPHQALLH TANRPEDECV
GEGLACHQLC ARGHCWGPGP TQCVNCSQFL RGQECVEECR VLQGLPREYV NARHCLPCHP
ECQPQNGSVT CFGPEADQCV ACAHYKDPPF CVARCPSGVK PDLSYMPIWK FPDEEGACQP
CPINCTHSCV DLDDKGCPAE QRASPLTEPE A.
NE12. A VLP (virus-like particle) monomer subunit, which comprises the variant according to any one of the preceding numbered embodiments, wherein said variant is linked to a polypeptide comprising in the N to C direction the following sub-elements a and b:
   a. a bait domain consisting of amino acid sequence SEQ ID NO: 6:
      GNPLIVMVND TTKVK, and
   b. a VLP monomer coat protein,
   wherein a and b are joined via linker amino acid residues or a bond.
NE13. The VLP monomer subunit according to numbered embodiment NE12, wherein a and b are joined via linker amino acid residues.
NE14. The VLP monomer subunit according to numbered embodiment NE13, wherein the linker amino acid residues constitutes amino acid sequence SEQ ID NO: 7:
   GSGTAGGGSG S.
NE15. The VLP monomer subunit according to any one of numbered embodiments NE12-NE14, wherein VLP monomer coat protein comprises or consists of amino acid sequence SEQ ID NO: 8: 
ANKPMQPITS TANKIVWSDP TRLSTTFSAS LLRQRVKVGI AELNNVSGQY VSVYKRPAPK
PEGCADACVI MPNENQSIRT VISGSAENLA TLKAEWETHK RNVDTLFASG NAGLGFLDPT
AAIVSSDTTA
NE16. The VLP monomer subunit according to any one of numbered embodiments NE12-NE15, wherein the variant and the polypeptide are linked via an isopeptide bond between the bait domain and the catcher domain.
NE17. The VLP monomer subunit according to numbered embodiment NE16, wherein the isopeptide bond links a K residue in the catcher domain and an N or D residue in the bait domain.
NE18. The VLP monomer subunit according to numbered embodiment NE17, wherein the K residue in the catcher domain is residue 32 in SEQ ID NO: 3, corresponding to residue 32 in SEQ ID NO: 1.
NE19. The VLP monomer subunit according to numbered embodiment NE17 or NE18, wherein the N or D residue in the bait domain is the N in position 9 in SEQ ID NO: 6, corresponding to the N in position 10 in SEQ ID NO: 5.
NE20. The VLP monomer subunit according to any one of numbered embodiments NE12-NE19, wherein the polypeptide has amino acid sequence SEQ ID NO: 5: 
MGNPLIVMVN DTTKVKGSGT AGGGSGSANK PMQPITSTAN KIVWSDPTRL STTFSASLLR
QRVKVGIAEL NNVSGQYVSV YKRPAPKPEG CADACVIMPN ENQSIRTVIS GSAENLATLK
AEWETHKRNV DTLFASGNAG LGFLDPTAAI VSSDTTA
NE21. The VLP monomer subunit according to any numbered embodiment NE12, which consists of the variant of SEQ ID NO: 1 linked via an isopeptide bond to the polypeptide of SEQ ID NO: 5, where the isopeptide bond links K in position 32 in SEQ ID NO: 1 with N in position 10 in SEQ ID NO: 5.
NE22. A virus like particle (VLP) composed of components i and ii:
   i) a plurality of VLP monomer subunits according to any one of numbered embodiments NE12-NE21, and
   ii) a plurality of polypeptides composed of sub-elements a and b defined in any one of numbered embodiments NE12-NE15,
   wherein the VLP monomer subunits preferably are according to numbered embodiment NE21 and polypeptides are according to numbered embodiment NE20.
NE23. The VLP according to numbered embodiment NE22, wherein the total number of components i and ii is 180.
NE24. The VLP according to numbered embodiment NE23, wherein about 50 of the total number are component i.
NE25. The VLP according to any one of numbered embodiments NE22-NE24, which has a molecular weight of about 7 MDa.
NE26. A composition comprising the VLP according to any one of numbered embodiments NE22-NE25 in admixture with a pharmaceutically acceptable vehicle, carrier, diuent, or excipient.
NE27. The composition according to numbered embodiment NE26, which in addition to the VLP comprises at least one buffering agent, and at least one tonicifying agent.
NE28. The composition according to numbered embodiment NE26 or NE27, which is in dry form, preferably in lyophilized form.
NE29. The composition according to numbered embodiment NE26 or NE27, which is an aqueous composition.
NE30. The composition according to numbered embodiment NE29, which comprises an immunological adjuvant.
NE31. The composition according to numbered embodiment NE30, wherein the immunological adjuvant is a water-in-oil emulsion adjuvant, preferably in the form of a mineral oil in admixture with a mannide monooleate surfactant.
NE32. The composition according to numbered embodiment NE31, wherein the adjuvant is Montanide ISA 51.
NE33. The composition according to numbered embodiment NE26, wherein the composition comprises water in admixture with 3.0 mg/ml of the VLP, 8.0 mg/ml NaCl, 0.2 mg/ml KCl, 0.2 mg/ml KH₂PO₄, 1.4 mg/ml Na₂PO₄.2H₂O, 1.2 mg/ml tris(hydroxymethyl)aminomethane, and 68.46 mg/ml sucrose.
NE34. A nucleic acid molecule encoding the variant according to any one of numbered embodiments NE1-NE11.
NE35. The nucleic acid molecule according to numbered embodiment NE34, which is a DNA molecule.
NE36. The nucleic acid molecule according to numbered embodiment NE35, which comprises residues 201-2453 in SEQ ID NO: 10.
NE37. The nucleic acid molecule according to numbered embodiment NE35, which comprises residues 147-2453 in SEQ ID NO: 10.
NE38. An expression vector, which comprises and is capable of expressing the nucleic acid molecule according to any one of numbered embodiments NE34-NE37.
NE39. The expression vector according to numbered embodiment NE38, which is capable of being expressed in a *Drosophila* S2 cell.
NE40. The Expression vector according to numbered embodiment NE39, which is a plasmid having the nucleotide sequence SEQ ID NO: 10.
NE41. An insect cell or cell line, which is transfected with the vector according to any one of numbered embodiments NE38-NE40.
NE42. The insect cell or cell line according to numbered embodiment NE41, which is stably transfected with the vector.
NE43. The insect cell or cell line according to numbered embodiment NE41 or NE42, which is a Drosophila cell or cell line, preferably an S2 cell or cell line.
NE44. A method of preparing the variant according to any one of numbered embodiments NE1-NE11, the method comprising culturing the insect cell or cell line according to any one of numbered embodiments NE41-NE43 under conditions that facilitate expression of the nucleic acid of any one of numbered embodiments NE34-NE37, and subsequently recovering and optionally purifying the variant from the culture medium.
NE45. A method of preparing the VLP according to any one of numbered embodiments NE22-NE25, the method comprising admixing the variant according to any one of numbered embodiments NE1-NE11 with self-assembled VLP precursors consisting of 80 sub-elements a and b defined in any one of numbered embodiments NE12-NE15 under conditions that facilitate coupling between catcher and bait domains via isopeptide bond formation.
NE46. The method according to numbered embodiment NE45, comprising preparing the variant according to the method of NE44 and preparing the self-assembled VLP precursors in a bacterial expression system.
NE47. A method of immunizing a human subject so as to induce an immune response against HER-2, comprising administration of an immunologically effective amount of the VLP according to any one NE22-NE25, or the composition according to any one of NE26-NE33, to said human subject.
NE48. The method according to numbered embodiment NE47, wherein the human subject suffers from a HER-2 expressing cancer, such as a HER-2 expressing breast cancer.
NE49. The method according to numbered embodiment NE47 or NE48, wherein the subject is administered a priming administration followed by one or more booster administrations.

### EXAMPLE 1

### Recombinant production of HER-2 fusion proteins

### Materials and Methods

### Construct design - Mooncake-HER-2_ECD

The extracellular domain (ECD) of HER-2 includes amino acids 1-652 from RefSeq protein entry NP_004439. The first 21 amino acids form the endogenous secretion signal of HER-2, which is removed during protein expression.

To facilitate virus-like particle (VLP) attachment, the HER-2_ECD (amino acids 22-652) was fused at the N-terminus to the Mooncake domain (Adaptvac) using a double-glycine flexible linker. The mooncake domain catalyzes the formation of an isopeptide bond between itself and a Rumtrunk peptide (Adaptvac). In this project, the rumtrunk peptide is genetically fused to a VLP, allowing the VLP surface to be decorated with HER-2_ECD. The BiP secretion signal sequence SEQ ID NO: 9 (MKLCILLAVVAFVGLSLG), see ref. 3, was fused to the N-terminus of the Mooncake sequence to enable secretion of the Mooncake-HER-2 protein into the cell supernatant. For purification, a C-tag (Thermo Fisher) affinity tag with the sequence EPEA was appended directly to the C-terminus of the HER-2_ECD to allow capture of the protein using Capture Select C-TagXL resin (Thermo Fisher).

The nucleic acid sequence encoding the BiP-Mooncake-HER2_ECD-C-tag polypeptide (SEQ ID NO: 11) is set forth in SEQ ID NO: 10, residues 147-2453. SEQ ID NO: 10 is the entire sequence of the circular expression plasmid.

### Construct design - HER-2-6His ECD

The Bip sequence was added N-terminally, to amino acids 623-652 of HER-2_ECD. Six histidines were added directly to the C terminus of HER-2 to enable affinity capture on immobilised metal affinity chromatography (IMAC) resin.

### Construct design - HER-2_ECD-Strep

The Bip sequence was added N-terminally, to amino acids 23-652 of HER-2_ECD. The strep affinity tag sequence (WSHPQFEK, SEQ ID NO: 31) was added directly to the C-terminus of HER-2_ECD to enable affinity capture on Streptactin XT Sepharose chromatography resin (IBA Lifesciences).

### Construct design - Twin-Strep-HER-2_ECD

Bip and the twin-strep sequence SEQ ID NO: 12 (WSHPQFEKGGGSGGGSGGSAWSHPQFEK, IBA Lifesciences) was added N-terminally to amino acids 623-652 of HER-2_ECD. The twin-strep sequence enables affinity capture on Streptactin XT Sepharose chromatography resin (IBA Lifesciences).

### Construct design - E2C-HER-2_ECD

BiP and amino acids 383-450 of human Carboxypeptidase D (RefSeq: accession NM_001304.5) was added N-terminally to amino acids 623-652 of HER-2_ECD. For purification, a C-tag (Thermo Fisher) affinity tag with the sequence EPEA was appended directly to the C-terminus of the HER-2_ECD.

### Protein purification - C-tag affinity capture

The harvest was thawed in a water bath at room temperature. Using tangential flow filtration (TFF), the harvest was concentrated 6-10-fold by ultrafiltration (UF) and the buffer was exchanged by diafiltration (DF) over 10 turnover volumes (TOV) into a capture buffer containing 25mM Tris, 150 mM NaCl, pH 7.5. The sample was then filtered using a 0.2 µM polyethersulfone (PES) sterile filter and loaded onto a 5mL CaptureSelect C-tagXL (Thermo Fisher) column for C-tag capture. The column was washed to baseline OD₂₈₀nm absorption in the same buffer. HER-2 protein was step-eluted in 25mM Tris pH 7.5 containing 500mM, 1000mM, and 2000mM MgCl2 while fractionating the eluate. The eluted protein was analyzed using SDS-PAGE, and fractions containing the protein of interest were pooled and concentrated on a 10kDa cutoff spin filter (Amicon, Millipore). For size exclusion chromatography (SEC) polishing, the sample was loaded on a SuperDex200pg 2.6/600 column (Cytiva) preequilibrated in 1 x Phosphate buffered Saline pH 7.4 (1 x PBS). Fractions containing HER-2 were analyzed using SDS-PAGE, and those containing the protein were pooled and concentrated again on a 10kDa cutoff spin filter before storage at - 80C.

### Protein purification - Strep tag affinity capture

Supernatant harvested from transiently or stably transfected S2 cells was subjected to tenfold ultrafiltration and ten turn over volumes (tov) diafiltration against Buffer W (IBA Lifesciences) using a tangential flow filtration (TFF) system (Centramate, 0.1 m2, 10 kDa molecular weight cut-off (MWCO) , Sartorius). The buffer-exchanged sample was loaded on a 5 ml StrepTactin XT column (IBA Biosciences), followed by column washing and eluted in fractions with 1xBXT Buffer (IBA Lifesciences) containing Biotin. Elution fractions were analyzed on Coomassie stained, reduced SDS-PAGE and those containing pure protein were pooled, buffer-exchanged to 1xPBS and concentrated using 30 kDa MWCO Amicon Ultra (Millipore) spin concentrators.

### Protein purification - IMAC capture

Supernatant was concentrated three-fold by ultrafiltration (UF) using a 10 kDa MWCO tangential filtration membrane. The protein was buffer exchanged into capture buffer by diafiltration using the same membrane and 10 turn-over-volumes. The protein was loaded onto 5 ml prepacked HisTrap HP (Cytiva) and eluted in step gradients at 12,5mM, 25mM, 50mM, 100mM, 250mM and 500mM Imidazole. Fractions containing the protein eluted at 100mM Imidazole and were pooled, concentrated using 30 kDa MWCO Amicon Ultra concentrator unit and separated on a HiLoad 16/600 Superdex 200 (Cytiva) gel filtration (GF) column equilibrated in PBS. Fractions containing the peak of HER-2 protein were pooled and concentrated on 30 kDa MWCO Amicon Ultra (Millipore) concentrator unit.

### Results

### Purification of Mooncake-HER-2_ECD

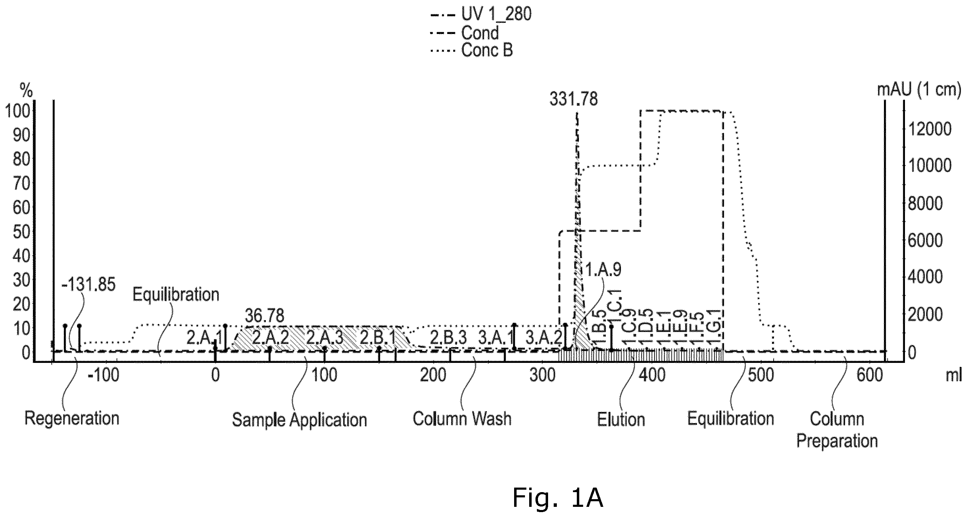

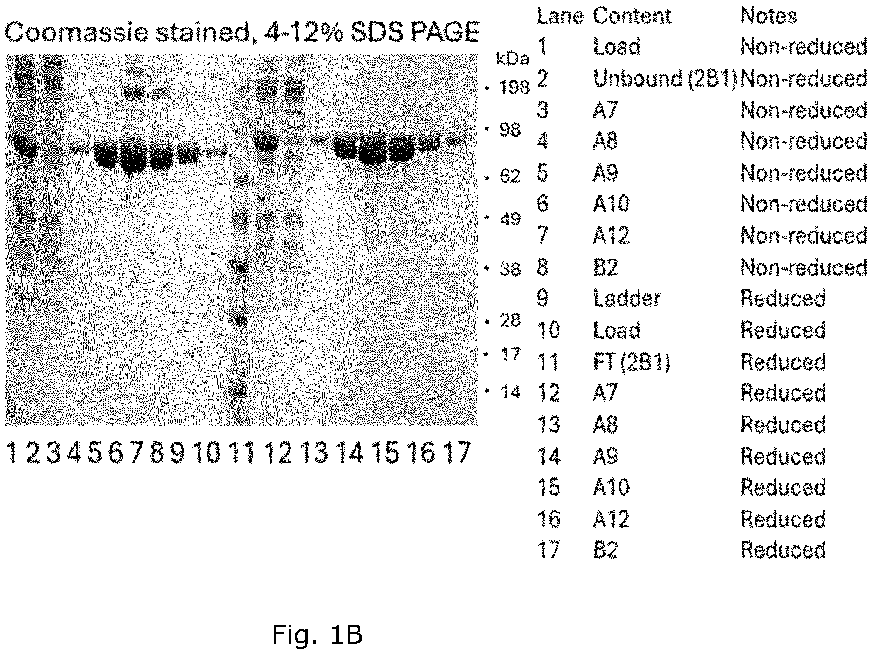

MoonCake-HER-2_ECD was captured on Capture Select C-tagXL resin (ThermoFisher). Chromatogram and SDS-PAGE analysis is shown in Fig. 1.

The purified yield of MoonCake-HER-2_ECD-C-tag was 75 mg purified protein per liter supernatant.

### Purification of HER-2_ECD for ELISA assay

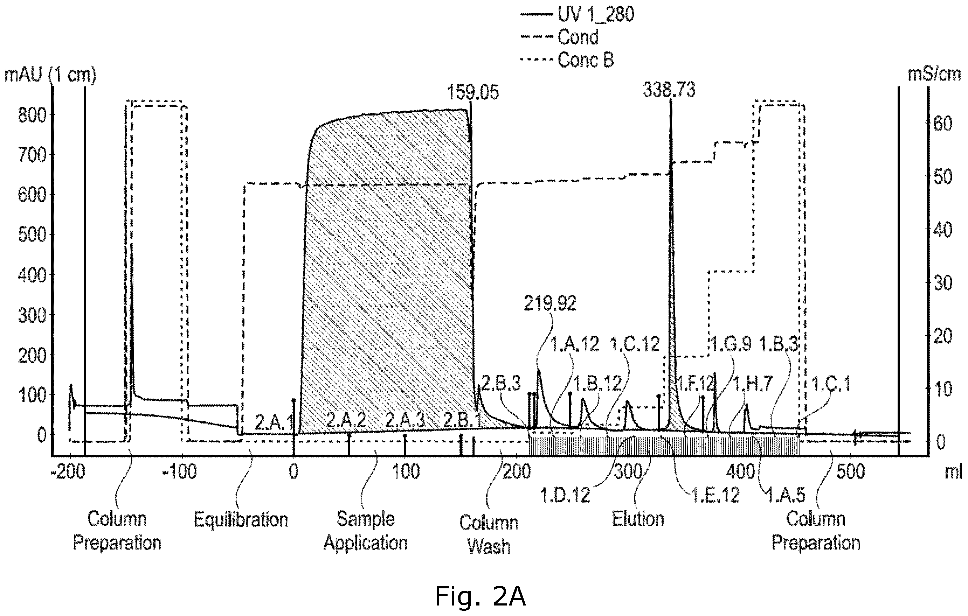

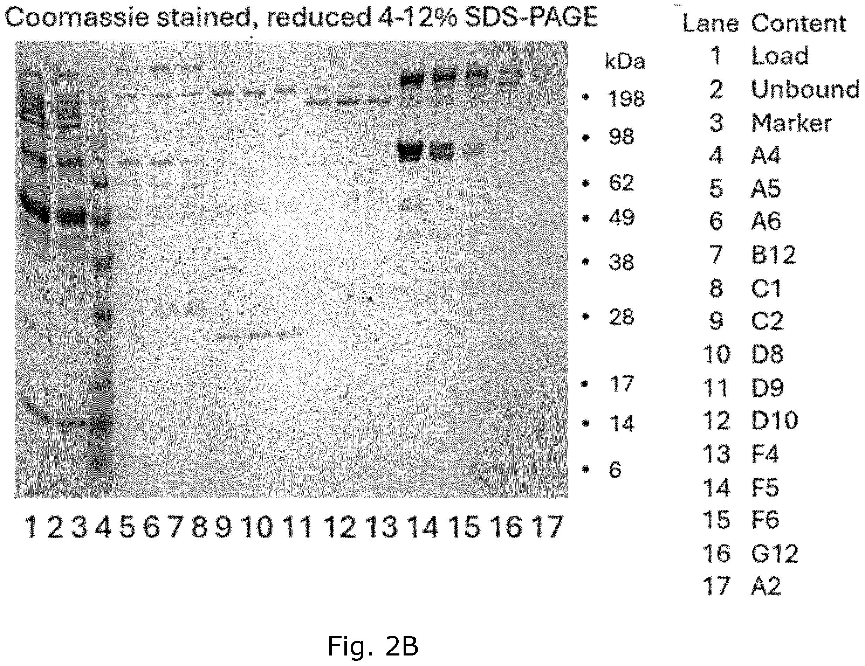

When used as a vaccine, immune responses would be expected to be directed against both MoonCake and HER-2_ECD. To exclusively determine the titre of anti-HER-2_ECD specific antibodies by ELISA assay, a new construct expressing the HER-2_ECD without MoonCake was needed. Initially, constructs were created where the MoonCake domain was removed from the HER-2_ECD N-terminal and the C-tag replaced with a 6xHIS tag. The constructs were used to express protein transiently in S2 cells and to select stable polyclonal pools expressing the HER-2_ECD-6xHIS protein. The purification and analysis of fractions from the purification of HER-2_ECD-6xHIS protein is illustrated in Fig. 2

The yield from IMAC capture was surprisingly low, 0.436 mg/l, while the MoonCake-HER-2_ECD yield had been 75 mg/l. As the fusion of a 6xHis tag to HER-2 would be expected to affect HER-2 expression less than fusion to the substantially larger MoonCake domain, the discrepancy was surprising.

By Trastuzumab immunoblot analysis of fractions F4 and F6 we determined the peak of HER-2_ECD-6xHis to be in fractions F4 - F6 (not shown). As the captured material was not as homogeneous (see Fig. 2, lanes 13 and 14) as obtained by C-tag capture, it was decided to replace the low selectivity 6xHis tag with the more selective Strep-tag (Iba lifesciences) and the 6xHs tag construct was hence changed to encode a single C-terminal streptag. The purification was performed as described for affinity capture of HER-2_ECD-6xHIS but the yield remained low, 2 mg/l.

### N terminal processing

The purified yields from HER-2_ECD constructs without MoonCake, were as noted only a minor fraction of the yields from the Mooncake-HER-2_ECD-C tag construct. Despite several attempts at re-batching, the yields were consistently significantly lower than expected. Scaling the volume 10× only decreased the specific yield from these cells.

The original MoonCake-HER-2_ECD-C tag construct was now modified in varous ways to include alternative affinity capture tags (C terminal 6 × HIS, or N terminal Twin Strep fusion). Also, a different folded domain called E2C was fused to the HER-2_ECD N-terminal; this significantly boosted yields of HER-2_ECD when expressed in the insect cells.

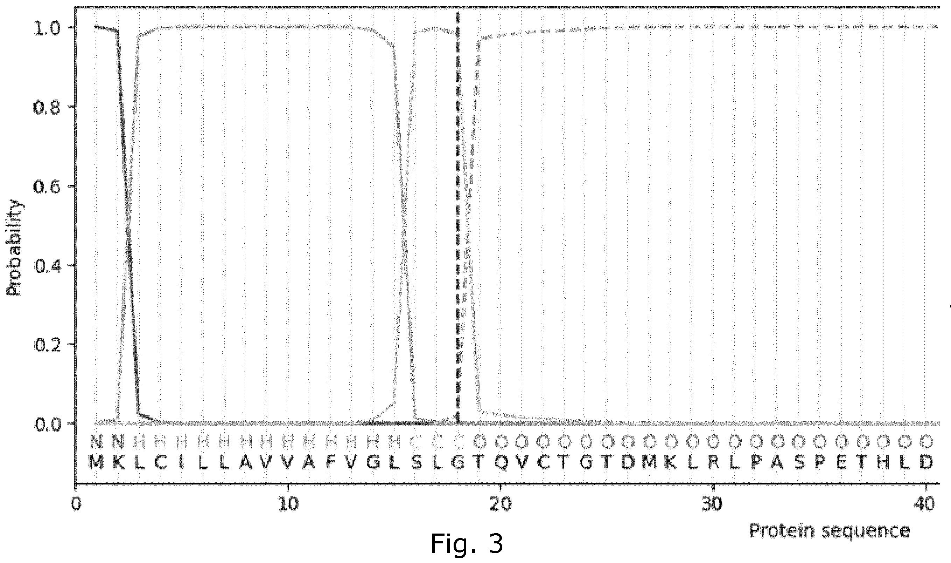

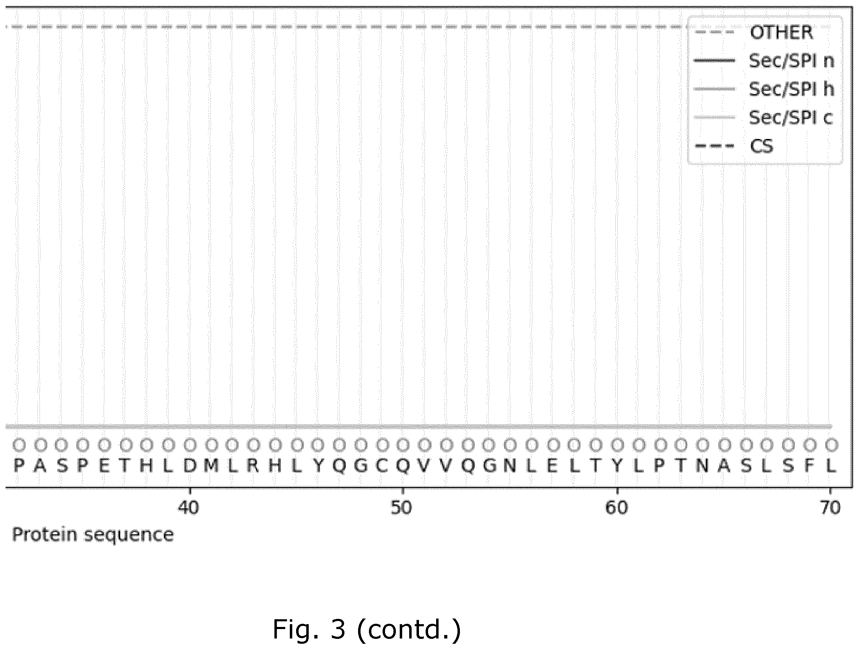

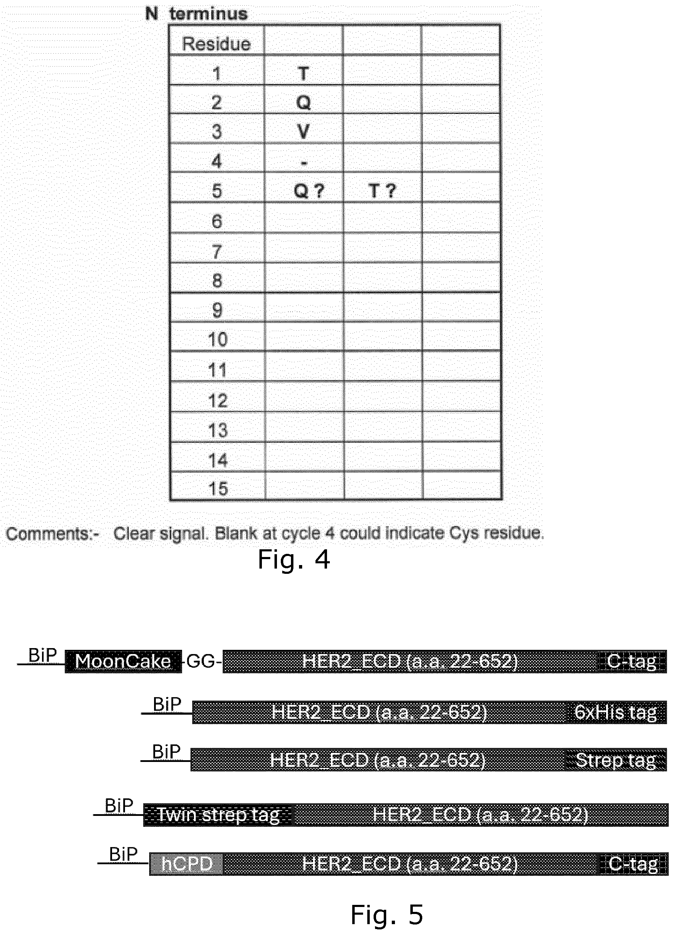

The endogenous HER-2_secretion peptide was then substituted with the BiP secretion signal to produce the protein in insect cells. During secretion BiP is removed by N-terminal processing by the cellular machinery. In some cases, and dependent on the protein, the processing results in heterogeneous N-termini, which could potentially destabilise the HER-2 protein fold. N-terminal sequencing was therefore of the expressed HER-2_ECD-Strep protein was therefore performed to investigate this potential probelm. As can be seen in Fig. 4, the N-terminal sequence corresponds exactly to the predicted sequence (as predicted with SignalP 6.0, see services.healthtech.dtu.dk/services/SignalP-6.0/) starting from amino acid 23. It was concluded that the HER-2 N-terminal is processed as would be expected from the primary sequence. Fig. 3 shows the predicted signal cleavage from SignalP 6.0 and Fig. 4 shows the result of N-terminal sequencing.

### N-terminal extension of HER-2 and the effect on expression levels

As the expression level of MoonCake-HER-2 was significantly higher than HER-2-Strep and HER-2-6×HIS, it was decided to test if N-terminal extension to HER-2 affected protein expression and/or stability.

MoonCake is a catalytic domain of 113 amino acids. N-terminal fusion of well-folded protein domains is commonly used to enhance solubility of proteins of eukaryotic origin when expressed in bacterial cells. The folding-enhancing abilities have been proposed to increase recruitment of chaperones during or after translation. To investigate the importance of N-terminal fusion for expressing HER-2_ECD, we created new constructs encoding HER-2 with either a 67 amino acid domain (approximately half MoonCake size) from human carboxypeptidase D (hCPD) or a 28 amino acid N-terminal twin strep tag (approximately quarter MoonCake size). Both domains/tags have previously been demonstrated to express well in S2 cells.

### Purification of Twin-Strep-HER-2_ECD

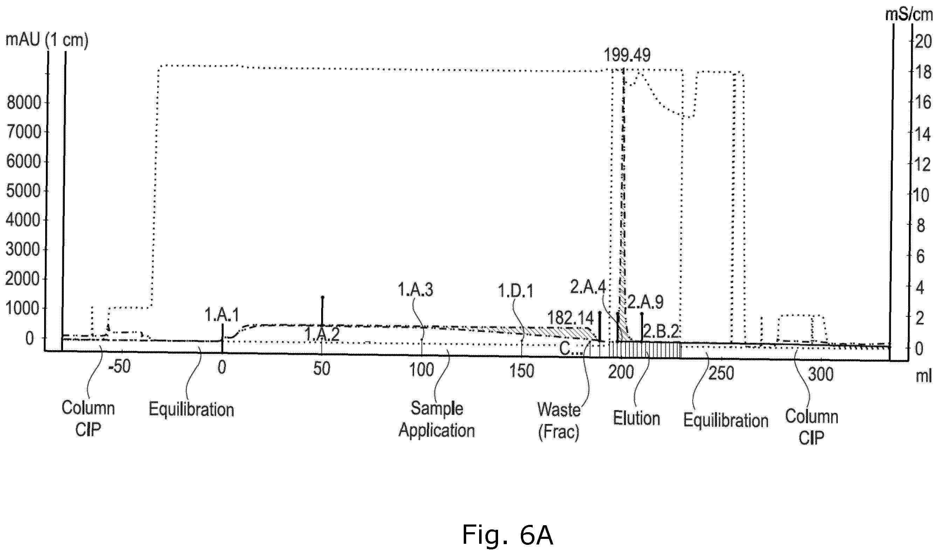

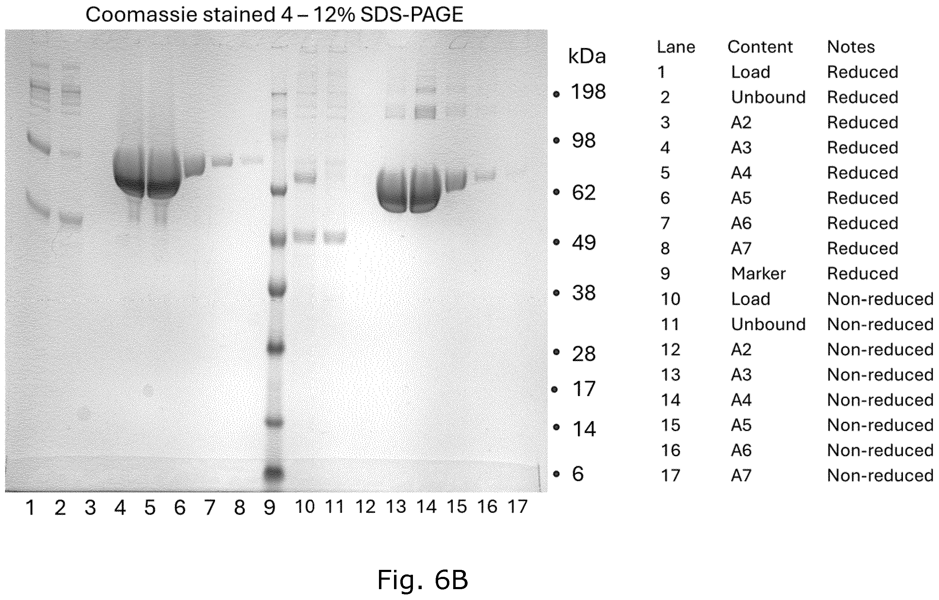

Transient transfection in large scale (250 ml) was used to produce Twin-Strep-HER-2_ECD. The protein was purified as described for strep tag purification above. The purification data is shown Fig. 6. After size exclusion chromatography (SEC) polishing, the final yield corresponded to 41.5 mg per litre (protocol EP-DW-415), significantly above the level for the previous constructs with no N-terminal extension to HER-2_ECD, but still well below the yield obtained with MoonCake_HER-2_ECD, cf. above.

### Purification of hCPD-HER-2_ECD-C

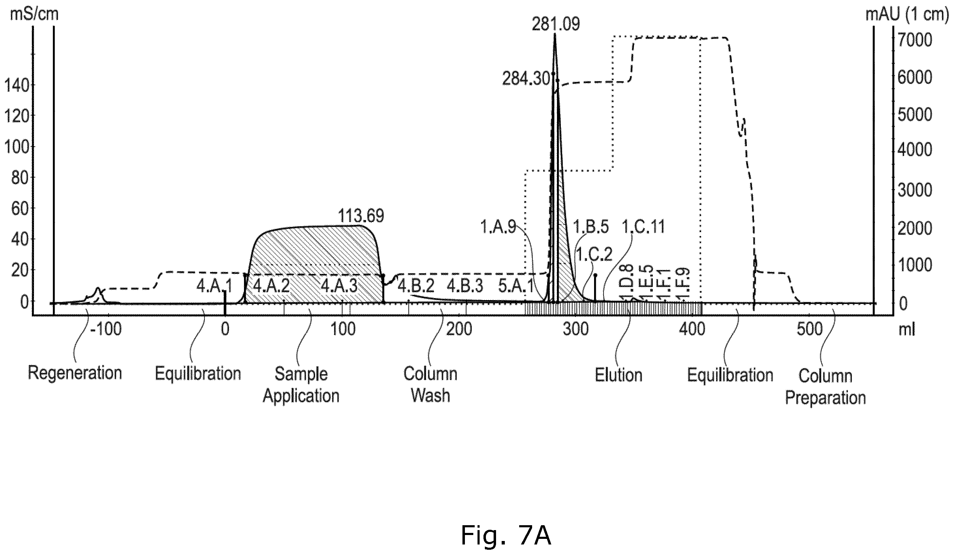

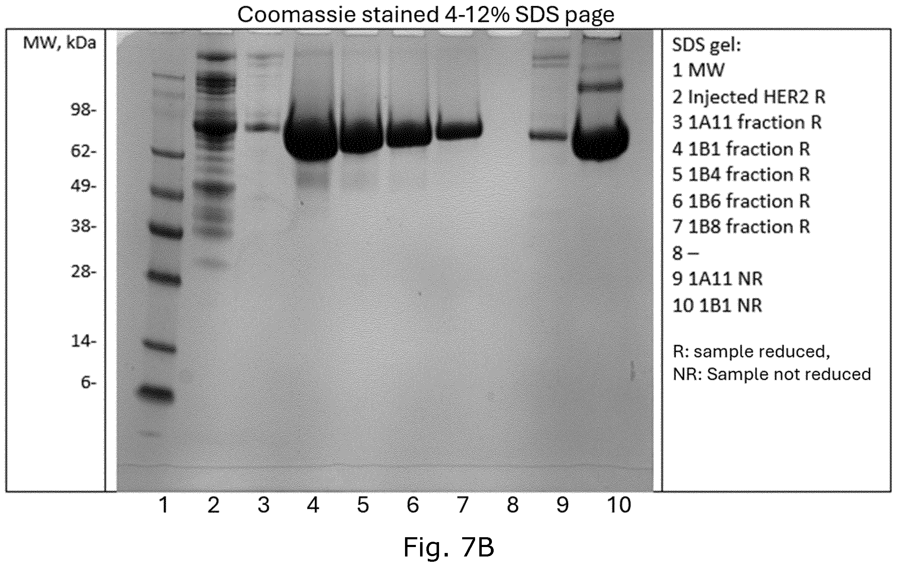

A stable polyclonal cell line expressing hCPD-HER-2_ECD-C tag was used to generate 900 ml supernatant. The purification was performed as described above for C tag purification. The final yield of purified protein corresponded to 88 mg/l. Results are presented in Fig. 7.

### Summary of results

The following table summarizes the expression yield results obtained with the tested HER-2 constructs:

| Construct | Harvest (I) | Yield (mg) | Yield conc. (mg/l) | Note |
|---|---|---|---|---|
| HER-2_strep | 1 | 2 | 2 | Transient |
| HER-2_strep | 1 | 1 | 1 | Transient |
| HER-2_strep | 1 | 0.4 | 0.4 | Stable |
| HER-2_strep | 10 | 11 | 1.1 | Stable |
| HER-2_strep | 1 | 2.35 | 2.35 | Stable |
| HER-2_strep | 10 | 14.62 | 1.462 | Stable |
| HER-2_6xHis | 0.5 | 0.218 | 0.436 | Transient |
| Twinstrep_HER-2 | 0.25 | 10.4 | 41.6 | Transient |
| hCPD-HER-2_ECD-C) | 1 | 88 | 88 | Stable |
| MoonCake-HER-2-Ctag | 1 | 75 | 75 | Stable |

### Discussion

The effect on expression levels of expressing human HER-2_ECD with different fusion partners at either terminus of HER-2 has been examined. It was found that recombinant HER-2 expressed quite poorly in S2 cells without having an N-terminal fusion partner. This is in itself surprising as natural HER-2 lacks an N-terminal fusion partner. Although literature suggests that proteins fold domain-wise (see ref. 4) and that the folding of one domain can facilitate the folding of a downstream domain (perhaps by spatial recruitment of chaperones), there does not appear to be any clear explanation why recombinant HER-2 ECD benefits from being fused N-terminally to a fusion partner.

Since the present technology relates to HER-2 VLP's this effect of N-terminal fusion on expression levels has been leveraged by fusing a catcher domain to the N-terminus of HER-2 ECD; this attains the dual goals of facilitating covalent coupling of HER-2 ECD to VLP monomer subunits and to ensure that recombinant production of HER-2 ECD results in high yields.

### LIST OF REFERENCES

1. Schneider, I. Cell lines derived from late embryonic stages of Drosophila melanogaster. J. Embryol. Exp. Morphol. 27, 353-365 (1972).
2. Fehr, T. et al. Role of repetitive antigen patterns for induction of antibodies against antibodies. J. Exp. Med. 185, 1785-1792 (1997).
3. Soejima, Y. et al. Comparison of signal peptides for efficient protein secretion in the baculovirus-silkworm system. Cent. Eur. J. Biol. 8, 1-7 (2013).
4. Han, Y. et al. Monitoring cotranslational protein folding in mammalian cells at codon resolution. Proc. Natl. Acad. Sci. U. S. A. 109, 12467-12472 (2012).

## Claims

1. A variant of HER-2 protein, which comprises the following amino acid sequence elements in N to C direction:
a. a catcher domain comprising or consisting of amino acid sequence SEQ ID NO: 3: 
GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHI
b. optionally one or more spacer amino acids,
c. a HER-2 extracellular domain, and
d. optionally an affinity tag.

2. The variant according to claim 1, which is essentially free from transmembrane and intracellular parts of HER-2.

3. The variant according to claim 1 or 2, wherein the HER-2 extracellular domain comprises or consists of amino acid sequence SEQ ID NO: 2: 
STQVCTGTDM KLRLPASPET HLDMLRHLYQ GCQVVQGNLE LTYLPTNASL SFLQDIQEVQ
GYVLIAHNQV RQVPLQRLRI VRGTQLFEDN YALAVLDNGD PLNNTTPVTG ASPGGLRELQ
LRSLTEILKG GVLIQRNPQL CYQDTILWKD IFHKNNQLAL TLIDTNRSRA CHPCSPMCKG
SRCWGESSED CQSLTRTVCA GGCARCKGPL PTDCCHEQCA AGCTGPKHSD CLACLHFNHS
GICELHCPAL VTYNTDTFES MPNPEGRYTF GASCVTACPY NYLSTDVGSC TLVCPLHNQE
VTAEDGTQRC EKCSKPCARV CYGLGMEHLR EVRAVTSANI QEFAGCKKIF GSLAFLPESF
DGDPASNTAP LQPEQLQVFE TLEEITGYLY ISAWPDSLPD LSVFQNLQVI RGRILHNGAY
SLTLQGLGIS WLGLRSLREL GSGLALIHHN THLCFVHTVP WDQLFRNPHQ ALLHTANRPE
DECVGEGLAC HQLCARGHCW GPGPTQCVNC SQFLRGQECV EECRVLQGLP REYVNARHCL
PCHPECQPQN GSVTCFGPEA DQCVACAHYK DPPFCVARCP SGVKPDLSYM PIWKFPDEEG
ACQPCPINCT HSCVDLDDKG CPAEQRASPL T,
 or an amino acid sequence, which has a sequence identity of at least 96% in a pairwise global alignment with SEQ ID NO: 2.

4. The variant according to any one of the preceding claims, wherein the C-terminal amino acid residue is the C-terminal amino acid residue in the affinity tag, such as the C-terminal A in SEQ ID NO: 4, and/or wherein the N-terminal amino acid residue is the N-terminal amino acid of the catcher domain.

5. The variant according to claim 1, which consists of the amino acid sequence SEQ ID NO: 1:

GIDTMSGLSG ETGQSGNTTI EEDSTTHVKF SKRDSNGKEL AGAMIELRNL SGQTIQSWVS
DGTVKDFYLM PGTYQFVETA APEGYELAAP ITFTVQDNGE VIIQGRLTRG DVHIGGSTQV
CTGTDMKLRL PASPETHLDM LRHLYQGCQV VQGNLELTYL PTNASLSFLQ DIQEVQGYVL
IAHNQVRQVP LQRLRIVRGT QLFEDNYALA VLDNGDPLNN TTPVTGASPG GLRELQLRSL
TEILKGGVLI QRNPQLCYQD TILWKDIFHK NNQLALTLID TNRSRACHPC SPMCKGSRCW

GESSEDCQSL TRTVCAGGCA RCKGPLPTDC CHEQCAAGCT GPKHSDCLAC LHFNHSGICE
LHCPALVTYN TDTFESMPNP EGRYTFGASC VTACPYNYLS TDVGSCTLVC PLHNQEVTAE
DGTQRCEKCS KPCARVCYGL GMEHLREVRA VTSANIQEFA GCKKIFGSLA FLPESFDGDP
ASNTAPLQPE QLQVFETLEE ITGYLYISAW PDSLPDLSVF QNLQVIRGRI LHNGAYSLTL
QGLGISWLGL RSLRELGSGL ALIHHNTHLC FVHTVPWDQL FRNPHQALLH TANRPEDECV
GEGLACHQLC ARGHCWGPGP TQCVNCSQFL RGQECVEECR VLQGLPREYV NARHCLPCHP
ECQPQNGSVT CFGPEADQCV ACAHYKDPPF CVARCPSGVK PDLSYMPIWK FPDEEGACQP
CPINCTHSCV DLDDKGCPAE QRASPLTEPE A.

6. A VLP (virus-like particle) monomer subunit, which comprises the variant according to any one of the preceding claims, wherein said variant is linked to a polypeptide comprising in the N to C direction the following sub-elements a and b:
a. a bait domain consisting of amino acid sequence SEQ ID NO: 6:
GNPLIVMVND TTKVK, and
b. a VLP monomer coat protein, such as SEQ ID NO: 8,
wherein a and b are joined via linker amino acid residues, such as SEQ ID NO: 7, or a bond.

7. The VLP monomer subunit according to claim 6, wherein the variant and the polypeptide are linked via an isopeptide bond between the bait domain and the catcher domain, such as an isopeptide bond, which links a K residue in the catcher domain and an N or D residue in the bait domain, in particular, wherein the K residue in the catcher domain is residue 32 in SEQ ID NO: 3, corresponding to residue 32 in SEQ ID NO: 1.

8. The VLP monomer subunit according to any one of claims 6-7, wherein the polypeptide has amino acid sequence SEQ ID NO: 5:

MGNPLIVMVN DTTKVKGSGT AGGGSGSANK PMQPITSTAN KIVWSDPTRL STTFSASLLR
QRVKVGIAEL NNVSGQYVSV YKRPAPKPEG CADACVIMPN ENQSIRTVIS GSAENLATLK
AEWETHKRNV DTLFASGNAG LGFLDPTAAI VSSDTTA

9. The VLP monomer subunit according to any claim 6, which consists of the variant of SEQ ID NO: 1 linked via an isopeptide bond to the polypeptide of SEQ ID NO: 5, where the isopeptide bond links K in position 32 in SEQ ID NO: 1 with N in position 10 in SEQ ID NO: 5.

10. A virus like particle (VLP) composed of components i and ii:
i) a plurality of VLP monomer subunits according to any one of claims 6-9, and
ii) a plurality of polypeptides composed of sub-elements a and b defined in any one of claims 6-9,
wherein the VLP monomer subunits preferably are according to claim 9 and polypeptides are according to claim 8.

11. The VLP according to claim 10, wherein the total number of components i and ii is 180 and wherein about 50 of the total number are component i, and which preferably has a molecular weight of about 7 MDa.

12. A composition comprising the VLP according to any one of claims 10-11 in admixture with a pharmaceutically acceptable vehicle, carrier, diuent, or excipient, and optionally at least one tonicifying agent.

13. The composition according to claim 12, which is in dry form, preferably in lyophilized form, or which is an aqueous composition.

14. The composition according to claim 13, which comprises an immunological adjuvant, preferably Montanide ISA 51.

15. A method of immunizing a human subject so as to induce an immune response against HER-2, comprising administration of an immunologically effective amount of the VLP according to any one 10-11, or the composition according to any one of claim 12-14, to said human subject, and wherein the human subject preferably suffers from a HER-2 expressing cancer, such as a HER-2 expressing breast cancer.
